# EUROPEAN PATENT SPECIFICATION

(11) **EP 1 689 876 B1**
(45) Date of publication and mention of the grant of the patent: **15.08.2012**
(21) Application number: 04801656.2
(22) Date of filing: 03.12.2004
(51) Int. Cl.: C12P 13/08, C12N 1/20, C12N 9/02

(54) **L-THREONINE PRODUCING BACTERIUM BELONGING TO THE GENUS ESCHERICHIA AND METHOD FOR PRODUCING L-THREONINE**
L-THREONIN PRODUZIERENDES BACTERIUM DER GATTUNG ESCHERICHIA UND METHODE ZUR HERSTELLUNG VON L-THREONIN
BACTERIE DU GENRE ESCHERICHIA PRODUISANT DE LA L-THREONINE ET METHODE POUR PRODUIRE CELLE-CI

(30) Priority: 05.12.2003 RU 2003135292; 09.07.2004 US 586222 P
(43) Date of publication of application: 16.08.2006
(73) Proprietor: Ajinomoto Co., Inc., Tokyo 104-8315 (JP)
(72) Inventor: AKHVERDIAN, Valery, Zavenovich, Moscow, 117593 (RU); SAVRASOVA, Ekaterina, Alekseevna, Moscow, 107370 (RU); KAPLAN, Alla, Markovna, Moscow, 107258 (RU); LOBANOV, Andrey, Olegovich, Moscow, 117465 (RU); KOZLOV, Yuri, Ivanovich, Moscow, 117574 (RU)
(74) Representative: HOFFMANN EITLE
(86) International application number: PCT/JP2004/018436
(87) International publication number: WO 2005/054490

(56) References cited:
- EP-A- 0 219 027
- WO-A-2004/108894
- DE-A1- 3 823 451
- US-A- 5 175 107
- US-B2- 7 723 097
- HAZIZA C ET AL: "NUCLEOTIDE SEQUENCE OF THE ASD GENE OF ESCHERICHIA-COLI ABSENCE OF A TYPICAL ATTENUATED SIGNAL" EMBO (EUROPEAN MOLECULAR BIOLOGY ORGANIZATION) JOURNAL, vol. 1, no. 3, 1982, pages 379-384, XP009044746 ISSN: 0261-4189
- VIOLA R E: "The central enzymes of the aspartate family of amino acid biosynthesis." ACCOUNTS OF CHEMICAL RESEARCH. MAY 2001, vol. 34, no. 5, May 2001 (2001-05), pages 339-349, XP002319930 ISSN: 0001-4842
- CHASSAGNOLE C ET AL: "An integrated study of threonine-pathway enzyme kinetics in Escherichia coli" BIOCHEMICAL JOURNAL, THE BIOCHEMICAL SOCIETY, LONDON, GB, vol. 356, no. 2, 1 June 2001 (2001-06-01), pages 415-423, XP002232684 ISSN: 0264-6021
- RAIS BADR ET AL: "Threonine synthesis from aspartate in Escherichia coli cell-free extracts: Pathway dynamics" BIOCHEMICAL JOURNAL, vol. 356, no. 2, 1 June 2001 (2001-06-01), pages 425-432, XP002319902 ISSN: 0264-6021

## Description

### Technical field

The present invention relates to a method for producing an L-threonine by fermentation, and more specifically to a gene derived from *Escherichia coli* which aids in this fermentation. The gene is useful for improvement of L-threonine production.

### Background art

Conventionally, L-amino acids are industrially produced by fermentation methods utilizing strains of microorganisms obtained from natural sources or mutants thereof, which are modified to enhance production yields of L-amino acids.

Many techniques to enhance production yields of L-amino acids have been reported, including transformation of microorganisms with recombinant DNA (see, for example, US patent No. 4,278,765). Other techniques for enhancing production yields include increasing the activities of enzymes involved in amino acid biosynthesis and/or desensitizing the target enzymes of the feedback inhibition by the resulting L-amino acid (see, for example, WO 95/16042 or US patent Nos. 4,346,170, 5,661,012 and 6,040,160).

Strains useful in production of L-threonine by fermentation are known, including strains with increased activities of enzymes involved in L-threonine biosynthesis (US patents 5,175,107; 5,661,012; 5,705,371; 5,939,307; EP0219027), strains resistant to chemicals such as L-threonine and its analogs (WO0114525A1, EP301572A2, US 5,376,538), strains with target enzymes desensitized to feedback inhibition by the produced L-amino acid or its byproducts (US patents 5,175,107; 5,661,012), and strains with inactivated threonine degradation enzymes (US patents 5,939,307; 6,297,031).

The known threonine-producing strain VKPM B-3996 (US patents 5,175,107, and 5,705,371) is the best threonine producer known at present. For construction of the strain VKPM B-3996, several mutations and a plasmid, described below, were introduced into parent strain *E. coli* K-12 (VKPM B-7). Mutant *thrA* gene (mutation *thrA*442) encodes aspartokinase homoserine dehydrogenase I, which is resistant to feedback inhibition by threonine. Mutant ilvA gene (mutation *ilvA*442) encodes threonine deaminase having decreased activity which results in a decreased rate of isoleucine biosynthesis and to a leaky phenotype of isoleucine starvation. In bacteria containing the *ilv*A442 mutation, transcription of the *thrABC* operon is not repressed by isoleucine, and therefore is very efficient for threonine production. Inactivation of the *tdh* gene results in prevention of threonine degradation. The genetic determinant of saccharose assimilation *(scrKYABR* genes) was transferred to said strain. To increase expression of the genes controlling threonine biosynthesis, plasmid pVIC40 containing mutant threonine operon *thrA442BC* was introduced in the intermediate strain TDH6. The amount of L-threonine accumulated during fermentation of the strain can be up to 85 g/l.

The present inventors obtained, with respect to *E*. *coli* K-12, a mutant, *thrR* (herein referred to as *rhtA*23) that has resistance to high concentrations of threonine or homoserine in minimal media (Astaurova, O.B. et al., Appl. Bioch. And Microbiol., 21, 611-616 (1985)). The mutation resulted in improvement in production of L-threonine (SU Patent No. 974817), homoserine, and glutamate (Astaurova, O.B. et al., Appl. Bioch. And Microbiol., 27, 556-561, 1991, EP 1013765 A) by the respective *E*. *coli* producing strain, such as the strain VKPM B-3996. Furthermore, the present inventors have revealed that the *rhtA* gene exists at 18 min on *E. coli* chromosome close to the *glnHPQ* operon that encodes components of the glutamine transport system, and that the *rhtA* gene is identical to ORF1 (*ybiF* gene, numbers 764 to 1651 in the GenBank accession number AAA218541, gi:440181), located between the *pexB* and *ompX* genes. The unit expressing a protein encoded by the ORF1 has been designated as *rhtA* (rht: resistance to homoserine and threonine) gene. Also, the present inventors have found that the *rhtA*23 mutation is an A-for-G substitution at position -1 with respect to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457, EP 1013765 A).

Under conditions of optimization of the mainstream threonine biosynthetic pathway, further improvement of threonine-producing strains can be accomplished by supplementing the bacterium with increasing amounts of distant precursors of threonine, such as aspartate.

It is known that aspartate is a donor of carbon for synthesis of the amino acids of the aspartate family (threonine, methionine, lysine), and diaminopimelate, a compound constituent of the bacterial cell wall. These syntheses are performed by a complex pathway having several branch points and an extremely sensitive regulatory scheme. In the branch points (aspartate, aspartate semialdehyde, homoserine), there are as many isozymes as there are amino acids deriving from this biosynthetic step. The aspartokinase homoserine dehydrogenase I encoded by part of *thrABC* operon causes the first and third reactions of threonine biosynthesis. Threonine and isoleucine regulate the expression of aspartokinase homoserine dehydrogenase I, and threonine inhibits both activities to catalyze the above-described reactions *(*Escherichia coli and Salmonella, Second Edition, Editor in Chief: F.C.Neidhardt, ASM Press, Washington D.C., 1996).

The *asd* gene encodes aspartate-β-semialdehyde dehydrogenase (Asd; EC 1.2.1.11), which is a key enzyme in the biosynthetic pathways for lysine, methionine, threonine and diaminopimelate. Aspartate-β-semialdehyde dehydrogenase reversibly converts L-aspartyl-4-P to L-aspartate semialdehyde along with the reduction of NADP. The effect of amplification of the *asd* gene on production of L-lysine, an amino acid of aspartate family, by *E. coli* strain is disclosed (US patent 6,040,160). It has also been disclosed that aspartate-β-semialdehyde dehydrogenase could be useful for production of L-lysine, L-threonine and L-isoleucine by coryneform bacteria (EP 0219027 A).

However, there has been no report to date of using a bacterium belonging to the genus *Escherichia* with enhanced aspartate-β-semialdehyde dehydrogenase activity for the production of L-threonine.

### SUMMARY OF THE INVENTION

An object of present invention is to enhance the productivity of L-threonine-producing strains and to provide a method for producing L-threonine using these strains.

This aim was achieved by finding that the *asd* gene encoding aspartate-β-semialdehyde dehydrogenase cloned on a low copy vector enhances L-threonine production. Thus the present invention has been completed.

It is an object of the present invention to provide a method for producing L-thronine which comprises cultivating an L-threonine-producing bacterium belonging to *Escherichia coli* wherein said bacterium has been modified to enhance an activity of aspartate-β-semialdehyde dehydrogenase, as defined in claim 1.

The activity of aspartate-β-semialdehyde dehydrogenase is enhanced by increasing the expression of an aspartate-β-semialdehyde dehydrogenase gene.

Said activity of aspartate-β-semialdehyde dehydrogenase is enhanced in one embodiment by increasing the copy number of the aspartate-β-semialdehyde dehydrogenase gene.

It is a further object of the present invention to provide the bacterium as described above, wherein the copy number is increased by transformation of the bacterium with a vector containing the gene.

It is a further object of the present invention to provide the bacterium as described above, wherein the aspartate-β-semialdehyde dehydrogenase gene is derived from a bacterium belonging to the genus *Escherichia.*

It is a further object of the present invention to provide the bacterium as described above, wherein said aspartate-β-semialdehyde dehydrogenase gene encodes
a protein which comprises the amino acid sequence of SEQ ID NO: 2;

It is a reference aspect to provide the bacterium as described above, wherein the aspartate-β-semialdehyde dehydrogenase gene comprises a DNA selected from the group consisting of:
(a) a DNA which comprises a nucleotide sequence of nucleotides 1 to 1196 in SEQ ID NO: 1; and
(b) a DNA which is hybridizable with a nucleotide sequence of nucleotides 1-1196 in SEQ ID NO: 1, or a probe which can be prepared from said nucleotide sequence under stringent conditions, and encodes a protein having an activity of aspartate-β-semialdehyde dehydrogenase.
Said stringent conditions comprise those in which washing is performed at 60°C at a salt concentration of 1 x SSC and 0.1 % SDS, and for 15 minutes.

The bacterium as described above has been further modified to enhance expression of the following genes.
- the mutant *thrA* gene which codes for aspartokinase homoserine dehydrogenase I and is resistant to feedback inhibition by threonine;
- the *thrB* gene which codes for homoserine kinase;
- the *thrC* gene which codes for threonine synthase; and
- the *rhtA* gene which codes for a putative transmembrane protein.

It is a further object of the present invention to provide the bacterium as described above, wherein said bacterium has been modified to increase expression of said mutant *thrA* gene, said *thrB* gene, *said thrC* gene and said *rhtA* gene, as described in claim 1.

It is a further object of the present invention to provide a method for producing L-threonine which comprises cultivating the bacterium as described above in a culture medium to cause accumulatation of L-threonine in the culture medium, and collecting the L-threonine from the culture medium.

### DESCRIPTION OF THE PREFERRED EMBODIMENTS

In the present disclosure "L-threonine-producing bacterium" means a bacterium which has an ability to cause accumulation of L-threonine in a medium when the bacterium is cultured in the medium. The L-threonine-producing ability may be imparted or enhanced by breeding. The phrase "L-threonine-producing bacterium" as used herein also means a bacterium which is able to produce and cause accumulation of L-threonine in a culture medium in amount larger than a wild-type or parental strain of *E. coli*, such as *E. coli* K-12 strain.

The phrase "a bacterium belonging to the genus *Escherichia*" means that the bacterium is classified in the genus *Escherichia* according to the classification known to a person skilled in the art of microbiology. The microorganism belonging to the genus *Escherichia* as used in the present invention is *Escherichia coli (E. coli).*

The phrase "activity of aspartate-β-semialdehyde dehydrogenase" means an activity which catalyzes the reversible substrate-dependent reduction of NADP in the presence of phosphate or arsenate. Activity of aspartate-β-semialdehyde dehydrogenase can be measured by the method described by, for example, Preiss, J. et al (Curr. Microbiol., 7: 263-268 (1982)).

The phrase "modified to enhance an activity of aspartate-β-semialdehyde dehydrogenase" means that the activity per cell is higher than that of a non-modified strain, for example, a wild-type strain. Examples of such modifications are defined in claim 1. Furthermore, a wild-type strain that may be used for comparison purposes includes, for example, *Escherichia coli* K-12. As a result of enhancement of intracellular activity of aspartate-β-semialdehyde dehydrogenase, the amount of L-threonine accumulation in a medium increases.

Enhancement of aspartate-β-semialdehyde dehydrogenase activity in a bacterial cell is attained by enhancement of expression of a gene encoding aspartate-β-semialdehyde dehydrogenase.

As the gene coding for aspartate-β-semialdehyde dehydrogenase of *Escherichia coli, asd* gene has already been elucidated (nucleotide numbers 3572511 to 3571408 in the sequence of GenBank accession NC_000913.1, gi:16131307). Therefore, the *asd* gene can be obtained by PCR (polymerase chain reaction; refer to White, T.J. et al., Trends Genet., 5, 185 (1989)) utilizing primers prepared based on the nucleotide sequence of the gene. Genes coding for aspartate-β-semialdehyde dehydrogenase of other microorganisms can be obtained in a similar manner. The *asd* gene derived from *Escherichia coli* is exemplified by a DNA which encodes the following protein (A)

(A) a protein which has the amino acid sequence shown in SEQ ID NO: 2;

"Transformation of a bacterium with DNA encoding a protein" means introduction of the DNA into a bacterium, for example, by conventional methods. Transformation of this DNA will result in an increase in expression of the gene encoding the protein of present invention, and will enhance the activity of the protein in the bacterial cell.

Methods of gene expression enhancement include in one embodiment increasing the gene copy number. Introducing a gene into a vector that is able to function in a bacterium belonging to the genus *Escherichia* increases the copy number of the gene. Preferably, low copy vectors are used. Examples of low-copy vectors include but are not limited to pSC101, pMW118, pMW119, and the like. The term "low copy vector" is used for vectors, the copy number of which is up to 5 copies per cell. Methods of transformation include any known methods that have hitherto been reported. For example, a method of treating recipient cells with calcium chloride so as to increase permeability of the cells to DNA has been reported for *Escherichia coli* K-12 (Mandel, M. and Higa, A., J. Mol. Biol, 53, 159 (1970)) and may be used.

Enhancement of gene expression may also be achieved by introduction of multiple copies of the gene into a bacterial chromosome by, for example, a method of homologous recombination, Mu integration or the like. For example, one act of Mu integration allows to introduce into bacterial chromosome up to 3 copies of the gene.

Enhancement of gene expression may also be achieved according to another embodiment by placing the DNA of the present invention under the control of a potent promoter, namely the *lac* promoter, the *trp* promoter, the *trc* promoter, and the P_{R}, and the P_{L} promoters of lambda phage which are known as potent promoters. Use of a potent promoter can be combined with multiplication of gene copies.

In a reference aspect, the effect of a promoter can be enhanced by, for example, introducing a mutation into the promoter to increase a transcription level of a gene located downstream of the promoter. Furthermore, it is known that substitution of several nucleotides in the spacer between ribosome binding site (RBS) and the start codon, especially the sequences immediately upstream of the start codon, profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629, 1984). Previously, it was shown that the *rhtA23* mutation is an A-for-G substitution at the -1 position relative to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457). Therefore, it may be suggested that the *rhtA23* mutation enhances the *rhtA* gene expression and, as a consequence, increases the resistance to threonine, homoserine and some other substances transported out of cells.

In a further reference aspect, it is also possible to introduce a nucleotide substitution into a promoter region of the aspartate-β-semialdehyde dehydrogenase gene on the bacterial chromosome resulting in a stronger promoter function. The alteration of the expression control sequence can be performed, for example, in the same manner as the gene substitution using a temperature-sensitive plasmid, as disclosed in International Publication WO00/18935 and Japanese Patent Publication No. 1-215280.

Increasing the copy number of the aspartate-β-semialdehyde dehydrogenase gene can also be achieved by introducing multiple copies of the aspartate-β-semialdehyde dehydrogenase gene into the chromosomal DNA of the bacterium. In order to introduce multiple copies of the aspartate-β-semialdehyde dehydrogenase gene into bacterial chromosome, homologous recombination is carried out using a sequence whose multiple copies exist as targets in the chromosomal DNA. Sequences having multiple copies in the chromosomal DNA include, but are not limited to repetitive DNA, or inverted repeats existing at the end of a transposable element. Also, as disclosed in US Patent No. 5,595,889, it is possible to incorporate the aspartate-β-semialdehyde dehydrogenase gene into a transposon, and allow it to be transferred to introduce multiple copies of the gene into the chromosomal DNA.

Methods for preparation of plasmid DNA include, but are not limited to digestion and ligation of DNA, transformation, selection of an oligonucleotide as a primer and the like, or other methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

The bacterium of the present invention can be obtained by introduction of the aforementioned DNAs into bacterium which inherently has the ability to produce L- threonine. Alternatively, the bacterium of the present invention can be obtained by imparting an ability to produce L- threonine to the bacterium already containing the DNAs.

Examples of parent strains encompassed by the present invention include, but are not limited to the threonine-producing bacteria belonging to *Escherichia coli such* as *E*. *coli* strain TDH-6/pVIC40 (VKPM B-3996) (US Patent 5,175,107, US patent 5,705,371), *E*. *coli* strain NRRL-21593 (US Patent 5,939,307), *E*. *coli* strain FERM BP-3756 (US patent 5,474,918), *E. coli* strains FERM BP-3519 and FERM BP-3520 (US patent 5,376,538), *E. coli* strain MG442 (Gusyatiner et al., Genetika (in Russian), 14, 947-956 (1978)), *E. coli* strains VL643 and VL2055 (EP 1149911 A), and the like.

The strain TDH-6 is deficient in the *thrC* gene, as well as being sucrose-assimilative, and the *ilvA* gene has a leaky mutation. This strain has a mutation in the *rhtA* gene, which imparts resistance to high concentrations of threonine or homoserine. The strain B-3996 contains the plasmid pVIC40 which had been obtained by inserting *thrA*BC* operon including mutant *thrA* gene encoding aspartokinase homoserine dehydrogenase I which has substantially desensitized feedback inhibition by threonine into RSF1010-derived vector. The strain B-3996 was deposited on November 19, 1987 in All-Union Scientific Center of Antibiotics (Nagatinskaya Street 3-A, 113105 Moscow, Russian Federation) under the accession number RIA 1867. The strain was also deposited on April 7, 1987 in Russian National Collection of Industrial Microorganisms (VKPM) (Dorozhny proezd. 1, Moscow 113545, Russian Federation) under the accession number B-3996.

The bacterium of the present invention is further modified to enhance expression of the following genes as well as *asd* gene:
- the mutant *thrA* gene which codes for aspartokinase homoserine dehydrogenase I resistant to feed back inhibition by threonine;
- the *thrB* gene which codes for homoserine kinase;
- the *thrC* gene which codes for threonine synthase;

The bacterium is also modified to enhance the *rhtA* gene which codes for a putative transmembrane protein in addition to enhancement of *asd* gene:

The method for producing L-threonine of the present invention includes the steps of cultivating the bacterium of the present invention in a culture medium, allowing L-threonine to accumulate in the culture medium, and collecting L-threonine from the culture medium.

In the present invention, the cultivation, collection and purification of L-threonine from the medium and the like may be performed in a manner similar to conventional fermentation methods wherein L-threonine is produced using a microorganism.

A medium used for culture may be either a synthetic or natural medium, so long as the medium includes a carbon source and a nitrogen source and minerals and, if necessary, appropriate amounts of nutrients which the microorganism requires for growth. The carbon source may include various carbohydrates such as glucose and sucrose, and various organic acids. Depending on the mode of assimilation of the chosen microorganism, alcohol including ethanol and glycerol may be used. As the nitrogen source, various ammonium salts such as ammonia and ammonium sulfate, other nitrogen compounds such as amines, a natural nitrogen source such as peptone, soybean-hydrolysate, and digested fermentative microorganism are used. As minerals, potassium monophosphate, magnesium sulfate, sodium chloride, ferrous sulfate, manganese sulfate, calcium chloride, and the like are used. As vitamins, thiamine, yeast extract and the like are used. Additional nutrients can be added to the medium, if necessary. For example, if the microorganism requires isoleucine for growth (isoleucine auxotrophy), a sufficient amount of isoleucine can be added to the cultivation medium.

The cultivation is performed preferably under aerobic conditions such as a shaking culture, and stirring culture with aeration, at a temperature of 20 to 40°C, preferably 30 to 38°C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to accumulation of L-threonine in the liquid medium.

After cultivation, solids such as cells can be removed from the liquid medium by centrifugation or membrane filtration, and then L- threonine can be collected and purified by ion-exchange, concentration and crystallization methods.

### Examples

The present invention will be more concretely explained below with reference to the following non-limiting examples.

### Example 1: Cloning of asd gene from E. coli into pM vector

The *asd* gene was cloned from chromosomal DNA of the *E. coli* strain (K12 Mu cts62 Mud5005) (VKPM B-6804) obtained from Russian National Collection of Industrial Microorganisms (VKPM) (Dorozhny proezd. 1, Moscow 113545, Russian Federation). First, mini-Mu phage in the *E*. *coli* strain (K12 Mu cts62 Mud5005) (VKPM B-6804) was induced. Then, the set of obtained derivatives of plasmids pMud5005 containing parts of chromosome was used for transformation of *asd* strain SH 309. The strain SH 309 (VKPM B-3899) obtained from Russian National Collection of Industrial Microorganisms (VKPM) (Dorozhny proezd. 1, Moscow 113545, Russian Federation) has the following phenotype: F⁻ araD139 rpsL150 deoC1 ptsF25 relAl feb5301 rbsR ugpA704::Tn10 Del (argF - lac) U169 Del (mal - asd) Tet^{R} Str^{R}. The asd⁻ strain SH 309 cannot grow on L-medium and requires diaminopimelinic acid (DAPA) for growth. SH 309 asd⁺ clones harboring plasmid pMud5005-asd were selected on the L-medium. The plasmid pMud5005-asd was isolated and *Bam*HI-*Pst*I DNA fragment (1646 bp) containing *asd* gene was recloned into the plasmid pMW119 previously modified to substitute promoter P*_{lac}* by promoter P_{R}. Thus the plasmid pMW-asd containing the *asd* gene under the control of promoter P_{R} was constructed. The plasmid pMW-asd is compatible with plasmid pVIC40 (replicon pRSF1010), therefore the two plasmids pVIC40 and pMW-asd could be maintained in the bacteria simultaneously.

The pMW-asd plasmid was introduced into the streptomycin-resistant threonine producer *E*. *coli* strain B-3996. Thus, the strain B-3996(pMW-asd) was obtained.

### Example 2. Effect of the asd gene amplification on threonine production

Both *E*. *coli* strains B-3996 and B-3996(pMW-asd) were grown for 18-24 hours at 37 °C on L-agar plates containing streptomycin (100 µg/ml) and ampicillin (100 µg/ml). To obtain seed culture, the strain was grown on a rotary shaker (250 rpm) at 32°C for 18 hours in 20x200 mm test tubes containing 2 ml of L-broth with 4% sucrose. Then, the fermentation medium was inoculated with 0.1 ml (5%) seed material. The fermentation was performed in 2 ml of minimal medium for fermentation in 20x200 mm test tubes. Cells were grown for 24 hours at 32°C with shaking at 250 rpm.

After cultivation, an accumulated amount of L-threonine in the medium was determined by TLC. Sorbfil plates (Stock Company Sorbopolymer, Krasnodar, Russia) were developed with a mobile phase: propan-2-ol : acetone : water : 25% aqueous ammonia = 25 : 25 : 7 : 6 (v/v). A solution (2%) of ninhydrin in acetone was used as a visualizing reagent. The results are presented in Table 1.

The composition of the fermentation medium (g/l) is as follows:

| | |
|---|---|
| Sucrose | 40.0 |
| (NH₄)₂SO₄ | 10.0 |
| KH₂PO₄ | 1.0 |
| MgS0₄ · 7H₂O | 0.4 |
| FeSO₄ · 7H₂O | 0.02 |
| MnSO₄ · 5H₂O | 0.02 |
| Thiamine HCl | 0.0002 |
| Yeast extract | 1.0 |
| CaCO₃ | 20.0 |
| L-Isoleucine | 0.05 |

Sucrose and magnesium sulfate are sterilized separately. CaCO₃ is dry-heat sterilized at 180°C for 2 h. The pH is adjusted to 7.0. Antibiotic is introduced into the medium after sterilization.

While the invention has been described in detail with reference to preferred embodiments thereof, it will be apparent to one skilled in the art that various changes can be made, and equivalents employed, without departing from the scope of the invention. Table 1.

| Strain | OD₅₆₀ | Threonine, g/l |
|---|---|---|
| B3996/pMW-asd | 8.6 | 18.5 |
| | 8.4 | 18.3 |
| | 9.1 | 19.8 |
| | 9.5 | 19.2 |
| | 9.3 | 20.0 |
| | 8.9 | 18.6 |
| | 9.4 | 19.3 |
| | 9.0 | 19.3 |
| | 9.0 ± 0.4 | 19.1 ± 0.6 |
| B-3996 (control) | 9.3 | 18.6 |
| | 9.6 | 17.9 |
| | 10.5 | 17.9 |
| | 10.6 | 17.6 |
| | 9.8 | 17.8 |
| | 9.9 | 18.1 |
| | 10.2 | 18.0 |
| | 10.0 | 17.9 |
| | 10.0 ± 0.4 | 18.0 ± 0.3 |

### Industrial Applicability

L-Threonine can be efficiently produced.

## Claims

1. A method for producing L-threonine which comprises cultivating an L-threonine-producing bacterium belonging to *Escherichia coli,* wherein said bacterium has been modified to enhance the activity of aspartate-β-semialdehyde dehydrogenase by increasing expression of the aspartate-β-semialdehyde dehydrogenase gene, wherein said bacterium has been further modified to enhance expression of the mutant thrA gene which codes for aspartokinase homoserine dehydrogenase I and is resistant to feedback inhibition by threonine; the thrB gene which codes for homoserine kinase; the thrC gene which codes for threonine synthase; and the rhtA gene which codes for a protein which imparts resistance to homeserine and threonine, in a culture medium to cause accumulation of L-threonine in the culture medium, and collecting the L-threonine from the culture medium,
wherein the expressions of the genes are enhanced by placing the genes under the control of a potent promoter and/or increasing the copy number of the genes, wherein said promoter under which aspartate-ß-semialdehyde dehydrogenase gene is placed is selected from the group consisting of trp promoter, trc promoter, P_{R} promoter, and P_{L} promoter,
wherein said aspartate-ß-semialdehyde dehydrogenase gene encodes a protein which comprises the amino acid sequence of SEQ ID NO: 2.

2. The method according to claim 1, wherein the copy number is increased by transformation of the bacterium with a vector containing the gene.

3. The method according to claim 1, wherein said promoter under which the rhtA gene is placed has the rhtA23 mutation which is an A-for-G substitution at the -1 position relative to the ATG start codon.

4. The method according to claim 1, wherein said promoter under which the aspartate-ß-semialdehyde dehydrogenase gene is placed is a P_{R} promoter.

## Patentansprüche

1. Verfahren zur Herstellung von L-Threonin, umfassend das Kultivieren eines L-Threonin produzierenden Bakteriums, welches zu Escherichia coli gehört, wobei das Bakterium modifiziert wurde, um die Aktivität von Aspartat-β-Semialdehyd-Dehydrogenase zu erhöhen, indem die Expression des Aspartat-β-Semialdehyd-Dehydrogenasegens erhöht wird, wobei das Bakterium weiter modifiziert wurde zur Expressionserhöhung des mutierten thrA-Gens, welches für die Aspartokinase-Homoserin-Dehydrogenase I kodiert und resistent gegenüber der Feedback-Inhibierung durch Threonin ist; des thrB-Gens, welches für Homoserinkinase kodiert; des thrC-Gens welches für Threoninsynthase kodiert und des rhtA-Gens, welches für ein Protein kodiert, welches Resistenz gegen Homoserin und Threonin verleiht, in einem Kulturmedium, um die Anreicherung von L-Threonin in dem Kulturmedium zu verursachen und das Sammeln von L-Threonin aus dem Kulturmedium,
wobei die Expression der Gene erhöht ist, indem die Gene unter die Kontrolle eines starken Promotors gestellt werden und/oder indem die Kopienzahl der Gene erhöht ist, wobei der Promotor, unter welchen das Aspartat-β-Semialdehyd-Dehydrogenasegen gestellt ist, ausgewählt ist aus der Gruppe bestehend aus dem trp-Promotor, dem trc-Promotor, dem P_{R}-Promotor und dem P_{L}-Promotor,
wobei das Aspartat-β-Semialdehyd-Dehydrogenasegen für ein Protein, welches die Aminosäuresequenz der SEQ ID NO: 2 umfasst, kodiert.

2. Verfahren gemäß Anspruch 1, wobei die Kopienzahl durch Transformation des Bakteriums mit einem Vektor, welcher das Gen enthält, erhöht ist.

3. Verfahren gemäß Anspruch 1, wobei der Promotor, unter welchen das rhtA-Gen gestellt ist, die rhtA23-Mutation aufweist, welche eine A-für-G-Substitution an der -1 Position relativ zum ATG-Startkodon ist.

4. Verfahren gemäß Anspruch 1, wobei der Promotor, unter welchen das Aspartat-β-Semialdehyd-Dehydrogenasegen gestellt ist, ein P_{R}-Promotor ist.

## Revendications

1. Procédé de production de L-thréonine qui comprend la culture d'une bactérie produisant la L-thréonine appartenant à l'Escherichia coli, où ladite bactérie a été modifiée pour améliorer l'activité de l'aspartate-β-semialdéhyde déshydrogénase en augmentant l'expression du gène de l'aspartate-β-semi-aldéhyde déshydrogénase, où ladite bactérie a été en outre modifiée pour améliorer l'expression du gène thrA mutant qui encode l'aspartokinase homosérine déshydrogénase I et résiste à la rétroinhibition par la thréonine ; le gène thrB qui effectue un codage pour l'homosérine kinase ; le gène thrC qui effectue un codage pour la thréonine synthase ; et le gène rhtA qui effectue un codage pour une protéine qui transmet une résistance à la homosérine et à la thréonine, dans un milieu de culture pour provoquer l'accumulation de la L-thréonine dans le milieu de culture, et la collecte de la L-thréonine du milieu de culture,
où les expressions des gènes sont améliorées en plaçant les gènes sous la commande d'un promoteur puissant et/ou en augmentant le nombre de copies des gènes, où ledit promoteur sous lequel est placé le gène de l'aspartate-β-semi-aldéhyde déshydrogénase est choisi parmi le groupe constitué du promoteur trp, du promoteur trc, du promoteur P_{R}, et du promoteur P_{L},
où ledit gène de l'aspartate-β-semi-aldéhyde déshydrogénase encode une protéine qui comprend la séquence d'acides aminés SEQ ID NO: 2.

2. Procédé selon la revendication 1, dans lequel le nombre de copies est augmenté par transformation de la bactérie avec un vecteur contenant le gène.

3. Procédé selon la revendication 1, dans lequel ledit promoteur sous lequel est placé le gène rhtA présente la mutation rhtA23 qui est une substitution A-pour-G à la position -1 par rapport au codon d'initiation ATG.

4. Procédé selon la revendication 1, dans lequel ledit promoteur sous lequel est placé le gène de l'aspartate-β-semi-aldéhyde déshydrogénase est un promoteur P_{R}.


### SEQUENCE LISTING

<110> Ajinomoto Co., Inc.
<120> L-THREONINE PRODUCING BACTERIUM BELONGING T0 THE GENUS ESCHERICHIA
   AND METHOD FOR PRODUCING L-THREONINE
<130> C2540PC4231
<150> RU 2003135292
   <151> 2003-12-05
<150> US 60/586,222
   <151> 2004-07-09
<160> 2
<210> 1
   <211> 1104
   <212> DNA
   <213> Escherichia coli
<220>
   <221> CDS
   <222> (1)..(1104)
<400> 1
   atg aaa aat gtt ggt ttt atc ggc tgg cgc ggt atg gtc ggc tcc gtt      48
   Met Lys Asn Val Gly Phe Ile Gly Trp Arg Gly Met Val Gly Ser Val
   1               5                   10                  15
   ctc atg caa cgc atg gtt gaa gag cgc gac ttc gac gcc att cgc cct      96
   Leu Met Gln Arg Met Val Glu Glu Arg Asp Phe Asp Ala Ile Arg Pro
               20                  25                  30
   gtc ttc ttt tct act tct cag ctt ggc cag gct gcg ccg tct ttt ggc     144
   Val Phe Phe Ser Thr Ser Gln Leu Gly Gln Ala Ala Pro Ser Phe Gly
           35                  40                  45
   gga acc act ggc aca ctt cag gat gcc ttt gat ctg gag gcg cta aag     192
   Gly Thr Thr Gly Thr Leu Gln Asp Ala Phe Asp Leu Glu Ala Leu Lys
       50                  55                  60
   gcc ctc gat atc att gtg acc tgt cag ggc ggc gat tat acc aac gaa     240
   Ala Leu Asp Ile Ile Val Thr Cys Gln Gly Gly Asp Tyr Thr Asn Glu
   65                  70                  75                  80
   atc tat cca aag ctt cgt gaa agc gga tgg caa ggt tac tgg att gac     288
   Ile Tyr Pro Lys Leu Arg Glu Ser Gly Trp Gln Gly Tyr Trp Ile Asp
                   85                  90                  95
   gca gca tcg tct ctg cgc atg aaa gat gac gcc atc atc att ctt gac     336
   Ala Ala Ser Ser Leu Arg Met Lys Asp Asp Ala Ile Ile Ile Leu Asp
               100                 105                 110
   ccc gtc aat cag gac gtc att acc gac gga tta aat aat ggc atc agg     384
   Pro Val Asn Gln Asp Val Ile Thr Asp Gly Leu Asn Asn Gly Ile Arg
           115                 120                 125
   act ttt gtt ggc ggt aac tgt acc gta agc ctg atg ttg atg tcg ttg     432
   Thr Phe Val Gly Gly Asn Cys Thr Val Ser Leu Met Leu Met Ser Leu
       130                 135                 140
   ggt ggt tta ttc gcc aat gat ctt gtt gat tgg gtg tcc gtt gca acc     480
   Gly Gly Leu Phe Ala Asn Asp Leu Val Asp Trp Val Ser Val Ala Thr
   145                 150                 155                 160
   tac cag gcc gct tcc ggc ggt ggt gcg cga cat atg cgt gag tta tta     528
   Tyr Gln Ala Ala Ser Gly Gly Gly Ala Arg His Met Arg Glu Leu Leu
                   165                 170                 175
   acc cag atg ggc cat ctg tat ggc cat gtg gca gat gaa ctc gcg acc     576
   Thr Gln Met Gly His Leu Tyr Gly His Val Ala Asp Glu Leu Ala Thr
               180                 185                 190
   ccg tcc tct gct att ctc gat atc gaa cgc aaa gtc aca acc tta acc     624
   Pro Ser Ser Ala Ile Leu Asp Ile Glu Arg Lys Val Thr Thr Leu Thr
           195                 200                 205
   cgt agc ggt gag ctg ccg gtg gat aac ttt ggc gtg ccg ctg gcg ggt     672
   Arg Ser Gly Glu Leu Pro Val Asp Asn Phe Gly Val Pro Leu Ala Gly
       210                 215                 220
   agc ctg att ccg tgg atc gac aaa cag ctc gat aac ggt cag agc cgc     720
   Ser Leu Ile Pro Trp Ile Asp Lys Gln Leu Asp Asn Gly Gln Ser Arg
   225                 230                 235                 240
   gaa gag tgg aaa ggg cag gcg gaa acc aac aag atc ctc aac aca tct     768
   Glu Glu Trp Lys Gly Gln Ala Glu Thr Asn Lys Ile Leu Asn Thr Ser
                   245                 250                 255
   tcc gta att ccg gta gat ggt tta tgt gtg cgt gtc ggg gca ttg cgc     816
   Ser Val Ile Pro Val Asp Gly Leu Cys Val Arg Val Gly Ala Leu Arg
               260                 265                 270
   tgc cac agc cag gca ttc act att aaa ttg aaa aaa gat gtg tct att     864
   Cys His Ser Gln Ala Phe Thr Ile Lys Leu Lys Lys Asp Val Ser Ile
           275                 280                 285
   ccg acc gtg gaa gaa ctg ctg gct gcg cac aat ccg tgg gcg aaa gtc     912
   Pro Thr Val Glu Glu Leu Leu Ala Ala His Asn Pro Trp Ala Lys Val
       290                 295                 300
   gtt ccg aac gat cgg gaa atc act atg cgt gag cta acc cca gct gcc     960
   Val Pro Asn Asp Arg Glu Ile Thr Met Arg Glu Leu Thr Pro Ala Ala
   305                 310                 315                 320
   gtt acc ggc acg ctg acc acg ccg gta ggc cgc ctg cgt aag ctg aat     1008
   Val Thr Gly Thr Leu Thr Thr Pro Val Gly Arg Leu Arg Lys Leu Asn
                   325                 330                 335
   atg gga cca gag ttc ctg tca gcc ttt acc gtg ggc gac cag ctg ctg     1056
   Met Gly Pro Glu Phe Leu Ser Ala Phe Thr Val Gly Asp Gln Leu Leu
               340                 345                 350
   tgg ggg gcc gcg gag ccg ctg cgt cgg atg ctt cgt caa ctg gcg taa     1104
   Trp Gly Ala Ala Glu Pro Leu Arg Arg Met Leu Arg Gln Leu Ala
           355                 360                 365
<210> 2
   <211> 367
   <212> PRT
   <213> Escherichia coli
<400> 2
   Met Lys Asn Val Gly Phe Ile Gly Trp Arg Gly Met Val Gly Ser Val
   1               5                   10                  15
   Leu Met Gln Arg Met Val Glu Glu Arg Asp Phe Asp Ala Ile Arg Pro
               20                  25                  30
   Val Phe Phe Ser Thr Ser Gln Leu Gly Gln Ala Ala Pro Ser Phe Gly
           35                  40                  45
   Gly Thr Thr Gly Thr Leu Gln Asp Ala Phe Asp Leu Glu Ala Leu Lys
       50                  55                  60
   Ala Leu Asp Ile Ile Val Thr Cys Gln Gly Gly Asp Tyr Thr Asn Glu
   65                  70                  75                  80
   Ile Tyr Pro Lys Leu Arg Glu Ser Gly Trp Gln Gly Tyr Trp Ile Asp
                   85                  90                  95
   Ala Ala Ser Ser Leu Arg Met Lys Asp Asp Ala Ile Ile Ile Leu Asp
               100                 105                 110
   Pro Val Asn Gln Asp Val Ile Thr Asp Gly Leu Asn Asn Gly Ile Arg
           115                 120                 125
   Thr Phe Val Gly Gly Asn Cys Thr Val Ser Leu Met Leu Met Ser Leu
       130                 135                 140
   Gly Gly Leu Phe Ala Asn Asp Leu Val Asp Trp Val Ser Val Ala Thr
   145                 150                 155                 160
   Tyr Gln Ala Ala Ser Gly Gly Gly Ala Arg His Met Arg Glu Leu Leu
                   165                 170                 175
   Thr Gln Met Gly His Leu Tyr Gly His Val Ala Asp Glu Leu Ala Thr
               180                 185                 190
   Pro Ser Ser Ala Ile Leu Asp Ile Glu Arg Lys Val Thr Thr Leu Thr
                   195                     200                     205
   Arg Ser Gly Glu Leu Pro Val Asp Asn Phe Gly Val Pro Leu Ala Gly
       210                 215                     220
   Ser Leu Ile Pro Trp Ile Asp Lys Gln Leu Asp Asn Gly Gln Ser Arg
   225                 230                 235                     240
   Glu Glu Trp Lys Gly Gln Ala Glu Thr Asn Lys Ile Leu Asn Thr Ser
                   245                 250                     255
   Ser Val Ile Pro Val Asp Gly Leu Cys Val Arg Val Gly Ala Leu Arg
               260                 265                     270
   Cys His Ser Gln Ala Phe Thr Ile Lys Leu Lys Lys Asp Val Ser Ile
           275                 280                     285
   Pro Thr Val Glu Glu Leu Leu Ala Ala His Asn Pro Trp Ala Lys Val
       290                 295                     300
   Val Pro Asn Asp Arg Glu Ile Thr Met Arg Glu Leu Thr Pro Ala Ala
   305                 310                 315                     320
   Val Thr Gly Thr Leu Thr Thr Pro Val Gly Arg Leu Arg Lys Leu Asn
                   325                 330                     335
   Met Gly Pro Glu Phe Leu Ser Ala Phe Thr Val Gly Asp Gln Leu Leu
               340                 345                     350
   Trp Gly Ala Ala Glu Pro Leu Arg Arg Met Leu Arg Gln Leu Ala
           355                 360                     365